# EUROPEAN PATENT SPECIFICATION

(11) **EP 0 438 519 B1**
(45) Date of publication and mention of the grant of the patent: **06.05.1998**
(21) Application number: 89912292.3
(22) Date of filing: 13.10.1989
(51) Int. Cl.: C07K 7/02, C07K 14/595, A61K 38/16

(54) **THERAPEUTIC PEPTIDES**
PEPTIDE ALS ARZNEIMITTEL
PEPTIDES THERAPEUTIQUES

(30) Priority: 14.10.1988 US 257998; 09.12.1988 US 282328; 02.03.1989 US 317941; 07.07.1989 US 376555; 21.08.1989 US 397169
(43) Date of publication of application: 31.07.1991
(73) Proprietor: The Administrators of The Tulane Educational Fund, New Orleans Louisiana 70112 (US)
(72) Inventor: COY, David, H., New Orleans, LA 70115 (US); MOREAU, Jacques-Pierre, Upton, MA 01568 (US); TAYLOR, John, E., Upton, MA 01568 (US); KIM, Sun, Hyuk, Chestnut Hill, MA 02167 (US)
(74) Representative: Deans, Michael John Percy
(86) International application number: US8904616
(87) International publication number: WO9003980

(56) References cited:
- US-A- 4 207 311
- US-A- 4 331 661
- US-A- 4 613 586
- US-A- 4 803 261
- No further relevant documents have been disclosed.
- Am J. of Physiol, (Maryland, USA), issued 1986, (HEINZ-ERIAN et al.), "(D- phe12) bombesin analogues: a new class of bombesin receptor antagonists", pages G 439-G442. See the Abstract in particular.
- Proc. Natl. Acad. Sci. USA (Washington, D.C., USA) Volume 82, issued November, 1985. (ZACHARY et al.), "High-affinity receptors for peptides of the bombesin family in Swiss 3T3 cells", pages 7616-7620. See Table 2, Neuromedin C and B in particular.
- Cancer Surveys (Oxford, England) Volume 4, No. 4, issued 1985 (CUTTITTA et al. ), "Autocrine growth factors in human small cell lung cancer", pages 707-727. See page 718 and Table 2 in particular.
- J. Med. Chem. (Washington, D.C., USA) Volume 30 issued 1987 (SASAKI et al.) "Solid-phase synthesis and biological Properties of psi(CH2NH) Pseudopeptide analogues of a highly Potent somatostatin octapeptide", pages 1162-1166. See pages 1162, 1164, 1166 in particular.
- CHEMICAL ABSTRACT, (Columbus, Ohio, USA) Volume 109, issued 1988, (COY et al.) "Probing peptide backbone function in bombesin. A reduced peptide bond analog
- with potent and specific receptor antagonist activity", the Abstract No. 32216K, J. Biol. Chem. 1988, 263 (11), 5056-60 (Eng).
- CHEMICAL ABSTRACT, (Columbus, Ohio, USA) Volume 109, issued 1988, (WOLL et al. ), "(Leu13-psi (CH2NH) Leu14) bombesin is a special bombesin receptor antagonist in Swiss 3T3 cells", the Abstract No 163928s, Biochem. Biophys. Res. Commun. 1988, 155(1), 359-65(Eng).

## Description

This invention relates to therapeutic peptides useful, e.g., for treatment of benign or malignant proliferation of tissue and for gastrointestinal disorders.

The amphibian peptide bombesin, pGlu-Gln-Arg-Leu-Gly-Asn-Gln-Trp-Ala-Val-Gly-His-Leu-Met-NH₂ (Anastasi et al., Experientia 27:166-167 (1971)), is closely related to the mammalian gastrin-releasing peptides (GRP), e.g., the porcine GRP, H₂N-Ala-Pro-val-Ser-Val-Gly-Gly-Gly-Thr-Val-Leu-Ala-Lys-Met-Tyr-Pro-Arg-Gly-Asn-His-Trp-Ala-Val-Gly-His-Leu-Met-(NH₂) (McDonald et al., Biochem. Biophys. Res. Commun. 90:227-233 (1979)) and human GRP, H₂N-Val-Pro-Leu-Pco-Ala-Gly-Gly-Gly-Thr-Val-Leu-Thr-Lys-Met-Tyr-Pro-Arg-Gly-Asn-His-Trp-Ala-Val-Gly-His-Leu-Met (NH₂). Bombesin has been found to be a growth factor for a number of human cancer cell lines, including small-cell lung carcinoma (SCLC), and has been detected in human breast and prostate cancer (Haveman et al., eds. Recent Results in Cancer Research - Peptide Hormones in Lung Cancer, Springer-Verlag, New York:1986). A number of these cancers are known to secrete peptide hormones related to GRP or bombesin. Consequently, antagonists to bombesin have been proposed as agents for the treatment of these cancers.

Pseudopeptide bombesin antagonists have been reported. See e.g., European Patent Application No. 309,297 (1989) (published after the priority date of the present application) and Coy, et al., J. Biol. Chem. 263(11):5056(1988). Peptide antagonists of bombesin have also been reported in which the C-terminal amino acid has been deleted. See e.g., European Patent Application Nos. 313,158 (1989) and 315,367 (1989) (published after the priority date of the present application). Bombesin agonists have also been reported. See e.g., U.S. Patent Nos. 4,331,661 (1982) and 4,207,311 (1980).

Cuttitta et al. demonstrated that a specific monoclonal antibody to bombesin inhibited in vivo the growth of a human small-cell lung cancer cell line xenografted to nude mice (Cuttitta et al., Cancer Survey 4:707-727 (1985)). In 3T3 murine fibroblasts which are responsive to the mitotic effect of bombesin, Zachary and Rozengurt observed that a substance P' antagonist (Spantide) acted as a bombesin antagonist (Zachary et al., Proc. Natl. Acad. Sci. (USA), 82:7616-7620 (1985)). Heinz-Erian et al. replaced His at position 12 in bombesin with D-Phe and observed bombesin antagonist activity in dispersed acini from guinea pig pancreas (Heinz-Erian et al., Am. J. of Physiol. 252:G439-G442 (1987)). Rivier reported work directed toward restricting the conformational freedom of the bioactive C-terminal decapeptide of bombesin by incorporating intramolecular disulfide bridges; however, Rivier mentioned that, so far, bombesin analogs with this modification fail to exhibit any antagonist activity (Rivier et al., "Competitive Antagonists of Peptide Hormones," in Abstracts of the International Symposium on Bombesin-Like Peptides in Health and Disease, Rome, Italy (October, 1987).

Abbreviations (uncommon) which are used herein:
pGlu = 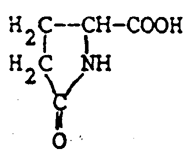 (pyroglutamatic acid);
Nle = H₂N-CH-COOH (norleucine)
   (CH₂)₃-CH₃
D-Cpa = D-p-chlorophenylalanine
HyPro = hydroxyproline
Nal = naphthylaianine
Sar = sarcosine

The invention provides a therapeutic peptide, said peptide comprising an analog of one of the following naturally occurring peptides terminating at the carboxy-terminus with a Met residue: (a) litorin, (b) neuromedin, (c) the ten amino acid carboxy-terminal region of mammalian gastrin releasing peptide, and (d) the ten amino acid carboxy-terminal region of amphibian bombesin; said peptide being of the formula: 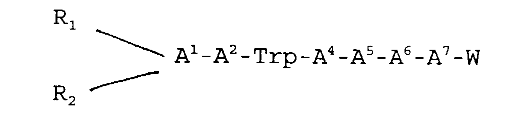 wherein
A¹ = the D-isomer of any of p-X-Phe (where X = F, Cl, Br, NO₂, OH, H or CH₃), Trp or β-Nal,
A² = Gln, His, 1-methyl-His, or 3-methyl-His,
A⁴ = Ala, Val, Gly, Leu, Ile, Nle or α-aminobutyric acid.
A⁵ = Gly, Ala, Leu, Ile, Nle, α-aminobutyric acid or Val,
A⁶ = Sar, Gly, or the D-isomer of any of Ala, Val, Leu or Ile,
A⁷ = 1-methyl-His, 3-methyl-His or His; and W is: 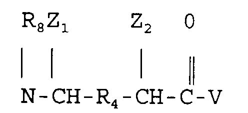 wherein R₄ is CH₂-NH, and each Z₁ and Z₂, independently, is the identifying group of any one of the amino acids Gly, Ala, Val, Leu, Ile, Ser, p-X-Phe (where X = H, F, C1, Br, NO₂, OH or CH₃), Trp, Met, Pro, HyPro, cyclohexyl-Ala, or cyclohexylmethyl and V is either OR₅ or 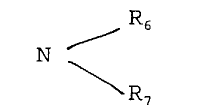 where each R₈, R₅, R₆, and R₇, independently, is H, lower alkyl, lower phenylalkyl, or lower naphthylalkyl; wherein any asymmetric carbon atom of the group W can be R, S or a racemic mixture; and wherein each R₁ and R₂, independently, is H, C₁₋₁₂ alkyl, C₇₋₁₀ phenylalkyl, COE₁ (where E₁ is C₁₋₂₀ alkyl, C₃₋₂₀ alkenyl, C₃₋₂₀ alkynyl, phenyl, naphthyl, or C₇₋₁₀ phenylalkyl), and R₁ and R₂ are bonded to the N-terminal amino acid of said peptide; provided that when one of R₁ or R₂ is COE₁, the other must be H;
   or a pharmaceutically acceptable salt thereof.

Preferably, the therapeutic peptide has the formula wherein A¹ = D-Phe, D-β-Nal or D-Cpa
A² = Gln, His, 1-methyl-His, or 3-methyl-His;
A⁴ = Ala;
A⁵ = Val;
A⁶ = Sar, Gly, or D-Ala;
A⁷ = His;
Z₁ is cyclohexylmethyl or is the identifying group of Leu or Phe; and Z₂ is the identifying group of Met, Leu or Phe.

Most preferably, the therapeutic peptide includes D-β-Nal at position A¹, where each of Z₁ and Z₂, independently, is Leu or Phe.

Examples of such peptides are: D-ß-Nal-Gln-Trp-Ala-Val-Gly-His-Leuψ[CH2NH]Leu-NH2, or D-ß-Nal-Gln-Trp-Ala-Val-Gly-His-Leuψ[CH2NH]Phe-NH2, wherein non-peptide bonds in which the peptide bond is reduced are symbolized by "ψ[CH₂NH]" or "ψ".

In addition, the carbon atom bonded to Z₂ is preferably of the R configuration. An example of such a peptide is

D-Phe-Gln-Trp-Ala-Val-Gly-His-Leuψ[CH2NH]-D-Phe-NH2.

Antagonists within the scope of the present invention are useful for treating diseases involving the malignant or benign proliferation of tissue, such as all forms of cancer where bombesin-related or GRP-related substances act as autocrine or paracrine mitotic factors, e.g., cancers of the gastrointestinal tract, pancreatic cancer, colon cancer, lung cancer, particularly the small cell subtype, or breast cancer; or for treating artherosclerosis, and disorders of gastrointestinal tissues related to gastric and pancreatic secretions and motility; for example, for causing the suppression of amylase secretion, or for appetite control.

In the qeneric formulae given above, when any one of R₁, R₂ or R₅-R₈ is an aromatic Lipophilic group, the in vivo activity can be long lasting, and delivery of the compounds to the target tissue can be facilitated.

The identifying group of an α-amino acid is the atom or group of atoms, other than the α-carbonyl carbon atom, the α-amino nitrogen atom, or the H atom, bound to the asymmetric α-carbon atom. To illustrate by examples, the identifying group of alanine is CH₃, the identifying group of valine is (CH₃)₂CH, the identifying group of lysine is H₃N⁺(CH₂)₄ and the identifying group of phenylalanine is (C₆H₅)CH₂.

Other features and advantages of the invention will be apparent from the following desorption of preferred embodiments.

We first briefly describe the drawings.

In the drawings:

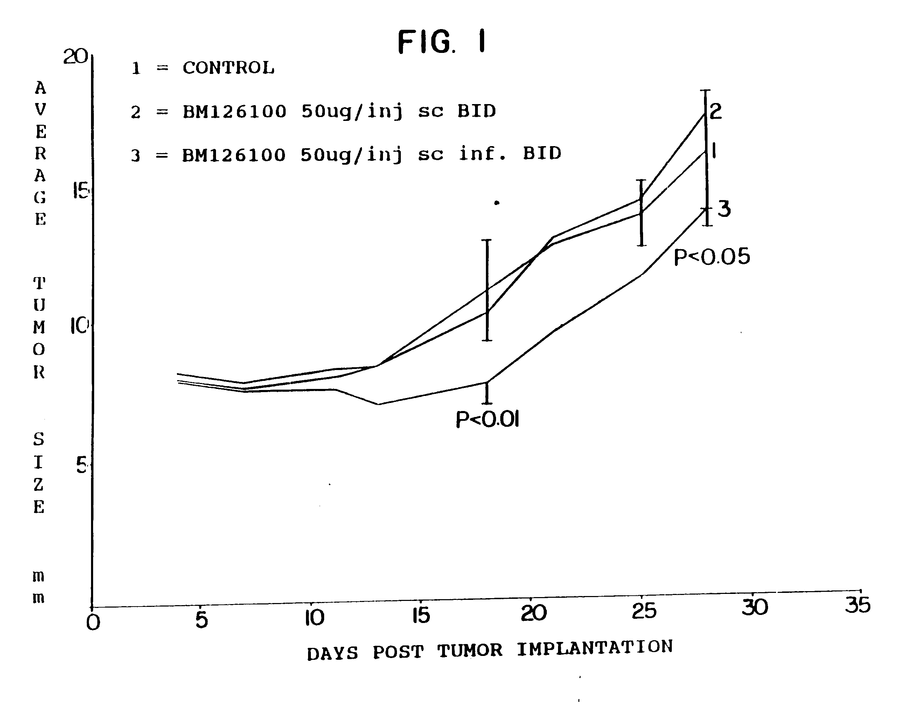

Fig. 1 is a graph of tumour growth curves for NCI-H69 xenografts.

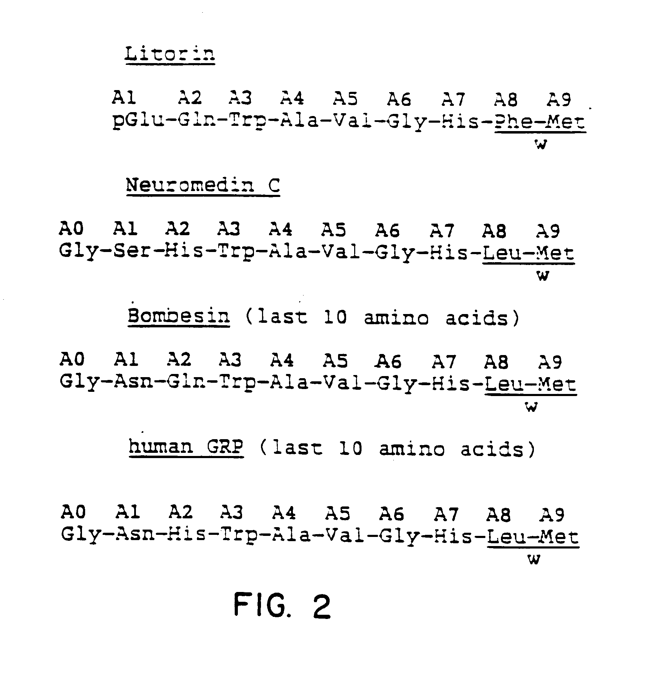

Fig. 2 is a series of amino acid sequences of naturally occurring peptides of which our present peptides are analogues.

Fig. 3 is a graph showing the effect of injection of the bombesin analogue D-Phe⁶BN(6-13)methylester on bombesin-stimulated pancreatic amylase assay.

We now describe the structure, synthesis, and use of preferred embodiments.

Our peptides all have a non-peptide bond. By non-peptide bond is meant that the carbon atom participating in the bond between two residues is reduced from a carbonyl carbon to a methylene carbon. The peptide bond reduction method which yields this non-peptide bond is described in Coy et al., U.S. patent Application, Serial No: 879,348.

Our peptides can be provided in the form of pharmaceutically acceptable salts.
Examples of preferred salts are those with therapeutically acceptable organic acids, e.g., acetic, lactic, maleic, citric, malic, ascorbic, succinic. benzoic, salicylic, methanesulfonic, toluenesulfonic, or pamoic acid, as well as polymeric acids such as tannic acid or carboxymethyl cellulose, and salts with inorganic acids such as the hydrohalic acids, e.g., hydrochloric acid, sulfuric acid, or phosphoric acid.

### Synthesis of Litorin Analogs

The synthesis of litorin antagonists of the formula A¹-Gln-Trp-Ala-Val-Gly-His-Leuψ[CH₂NH]Leu-NH₂ is as set out below. Other antagonists of bombesin, litorin, neuromedin, or GRP can be prepared by making appropriate modifications of the following synthetic method.

The first step is the preparation of the intermediate A¹-Gln-Trp-Ala-Val-Gly-His(benzyloxycarbonyl)-Leuψ[CH₂ NH]Leu-benzhydrylamine resin, as follows.

Benzhydrylamine-polystyrene resin (Vega Biochemicals, Inc.) (0.97 g, 0.5 mmole) in the chloride ion form is placed in the reaction vessel of a Beckman 990B peptide synthesizer programmed to perform the following reaction cycle: (a) methylene chloride; (b) 33% trifluoroacetic acid (TFA) in methylene chloride (2 times for 1 and 25 min. each); (c) methylene chloride; (d) ethanol; (e) methylene chIoride; and (f) 10% triethylamine in chloroform.

The neutralized resin is stirred with alpha-t-butoxycarbonyl(Boc)-leucine and diisopropylcarbodiimide (1.5 mmole each) in methylene chloride for 1 hour, and the resulting amino acid resin. is then cycled through steps (a) to (f) in the above wash program. Boc-leucine aldehyde (1.25 mmoles), prepared by the method of Fehrentz and Castro, Synthesis, p. 676 (1983), is dissolved in 5 ml of dry dimethylformamide (DMF) and added to the resin TFA salt suspension followed by the addition of 100 mg (2 mmoles) of sodium cyanoborohydride (Sasaki and Coy, Peptides 8:119-121 (1987); Coy et al., id.). After stirring for 1 hour, the resin mixture is found to be negative to ninhydrin reaction (1 min.), indicating complete derivatization of the free amino group.

The following amino acids (1)5 mmole) are then coupled successively in the presence diisopropylcarbodiimide (1.5 mmole), and the resulting amino acid resin is cycled through washing/deblocking steps (a) to (f) in the same procedure as above: Boc-His(benzyloxycarbonyl), Boc-Gly, Boc-Val, Boc-Ala, Boc-Trp. Boc-Gln (coupled as a 6 M excess of the p-nitrophenylester), and then the selected A¹. The completed resin is then washed with methanol and air dried.

The resin described above is mixed with anisole (5 ml) and anhydrous hydrogen fluoride (35 ml) at 0°C and stirred for 45 min. Excess hydrogen fluoride is evaporated rapidly under a stream of dry nitrogen, and free peptide is precipitated and washed with ether. The crude peptide is dissolved in a minimum volume of 2 M acetic acid and eluted on a column (2.5 x 100 mm) of Sephadex G-25 (Pharmacia Fine Chemicals, Inc.). Fractions containing a major component by uv absorption and thin layer chromatography (TLC) are then pooled, evaporated to a small volume and applied to a column (2.5 x 50 cm) of octadecylsilane-silica (Whatman LRP-1, 15-20 µm mesh size).

The peptide is eluted with a linear gradient of 0-30% acetonitrile in 0.1% trifluoroacetic acid in water. Fractions are examined by TLC and analytical high performance liquid chromatography (HPLC) and pooled to give maximum purity. Repeated lyophilization of the solution from water gives the product as a white, fluffy powder.

The product can be found to be homogeneous by HPLC and TLC. Amino acid analysis of an acid hydrolysate confirms the composition of the peptide. The presence of the Leuψ[CH₂-NH]Leu bond is demonstrated by fast atom bombardment mass spectrometry. Similar peptides such as A'-Gln-Trp-Ala-Val-Gly-His-Pheψ[CH₂NH]Leu-NH₂ or other peptides are prepared in similar yields in an analogous fashion by appropriately modifying the above procedure.

Solid phase synthesis of D-Phe¹, Leu⁸ψ[CH₂NH]D-Phe⁹-litorin, D-Phe-Gln-Trp-Ala-Val-Gly- His-Leuψ[CH₂NH]-D-Phe-NH₂, was carried out as follows:
Boc-D-Phe-Gln-Trp-Ala-Val-Gly-His(tosyl)-Leuψ[CH₂NH]-D-Phe-benzydrylamine resin Was synthesized first.

Benzhydrylamine-polystyrene resin (Advanced ChemTech, Inc.) (1.25 g, 0.5 mmole) in the chloride ion form is placed in the reaction vessel of an Advanced ChemTech ACT 200 peptide synthesizer programmed to perform the reaction cycle described as steps (a) through (f) above.

The neutralized resin is stirred with Boc-D-phenylalanine and diisopropylcarbodiimide (1.5 mmole each) in methylene chloride for 1 h and the resulting amino acid resin is then cycled through steps (a) to (g) in the above wash program. The Boc group is then removed by TFA treatment. Boc-leucine aldehyde (1.25 mmoles), prepared by the method of Fehrentz and Castro (1), is dissolved in 5 ml of dry DMF and added to the resin TFA salt suspension followed by the addition of 100 mg (2 mmoles) of sodium cyanoborohydride (2,3). After stirring for 1 h, the resin mixure is found to be negative to ninhydrin reaction (1 min) indicating complete derivatization of the free amino group.

The following amino acids (1.5 mmole) are then coupled successively by the same procedure:
Boc-His(benzyloxycarbonyl), Boc-Gly, Boc-Val, Boc-Ala, Boc-Trp, Boc-Gln (coupled in the presence of 1 equiv. hydroxybenzotriazole), Boc-D-Phe (coupled in the presence of 1 equiv. hydroxybenzotriazole). After drying, the peptide resin weighed 1.93 g.

The resin (1.93 g, 0.5 mmole) is mixed with anisole (5 ml) and anhydrous hydrogen fluoride (35 ml) at 0°C and stirred for 45 min. Excess hydrogen fluoride is evaporated rapidly under a stream of dry nitrogen and free peptide precipitated and washed with ether. The crude peptide is dissolved in a minimum volume of 2 M acetic acid and eluted on a column (2.5 x 100 mm) of Sephadex G-25. Fractions containing a major component by uv absorption and thin layer chromatography are then pooled, evaporated to a small volume and applied to a column (2.5 x 50 cm) of Vydac octadecylsilane (10-15 uM). This is eluted with a linear gradient of 15-45% acetonitrile in 0.1% trifluoroacetic acid in water. Fractions are examined by thin layer chromatography and analytical high performance liquid chromatography and pooled to give maximum purity. Repeated lyophlization of the solution from water gives 120 mg of the product as a white, fluffy powder.

Other peptides, e.g., D-p-Cl-Phe-Gln-Trp-Ala-Val-Gly-His-Leuψ[CH₂NH]-D-Phe-NH₂, may be synthesized using essential the same procedure.

### Synthesis of Leu⁸ψ[CH₂NH] D-Phe⁹ Litorin

Solid phase synthesis of D-Phe-Gln-Trp-Ala-Val-Gly-His-Leuψ[CH₂NH]-D-Phe-NH₂ was carried out as follows:
Boc-D-Phe-Gln-Trp-Ala-Val-Gly-His(tosyl)-Leuψ[CH₂NH]-D-Phe-benzhydrylamineresinwassynthesizedfirst.

Benzhydrylamine-polystyrene resin (Advanced ChemTech, Inc.) (1.25 g, 0.5 mmole) in the chloride ion form is placed in the reaction vessel of an Advanced ChemTech ACT 200 peptide synthesizer programmed to perform the following reaction cycle: (a) methyiene chloride; (b) 33% trifluoroacetic acid in methylene chloride (2 times for 1 and 25 min each); (c) methylene chloride; (d) ethanol; (e) methylene chloride; (f) 10% triethylamine in chloroform.

The neutralized resin is stirred with Boc-D-phenylalanine and diisopropylcarbodiimide (1.5 mmole each) in methylene chloride for 1 h and the resulting amino acid resin is then cycled through steps (a) to (g) in the above wash program. The Boc group is then removed by TFA treatment. Boc-leucine aldehyde (1.25 mmoles), prepared by the method of Fehrentz and Castro (1), is dissolved in 5 ml of dry DMF and added to the resin TFA salt suspension followed by the addition of 100 mg (2 mmoles) of sodium cyanoborohydride (2,3). After stirring for 1 h, the resin mixure is found to be negative to ninhydrin reaction (1 min) indicating complete derivatization of the free amino group.

The following amino acids (1.5 mmole) are then coupled successively by the same procedure: Boc-His(benzyloxycarbonyl), Boc-Gly, Boc-Val, Boc-Ala, Boc-Trp, Boc-Gln (coupled in the presence of 1 equiv. hydroxybenzotriazole), Boc-D-Phe (coupled in the presence of 1 equiv. hydroxybenzotriazole). After drying, the peptide resin weighed 1.93 g.

The resin (1.93 g, 0.5 mmole) is mixed with anisole (5 ml) and anhydrous hydrogen fluoride (35 ml) at 0°C and stirred for 45 min. Excess hydrogen fluoride is evaporated rapidly under a stream of dry nitrogen and free peptide precipitated and washed with ether. The crude peptide is dissolved in a minimum volume of 2 M acetic acid and eluted on a column (2.5 x 100 mm) of Sephadex G-25. Fractions containing a major component by uv absorption and thin layer chromatography are then pooled, evaporated to a small volume and applied to a column (2.5 x 50 cm) of Vydac octadecylsilane (10-15 uM). This is eluted with a linear gradient of 15-45% acetonitrile in 0.1% trifluoroacetic acid in water. Fractions are examined by thin layer chromatography and analytical high performance liquid chromatography and pooled to give maximum purity. Repeated lyophlization of the solution from water gives 120 mg of the product as a white, fluffy powder.

The product is found to be homogeneous by hplc and tlc. Amino acid analysis of an acid hydrolysate confirms the composition of the octapeptide. The presence of the Leuψ[CH₂NH] peptide bond is demonstrated by fast atom bombardment mass spectrometry.

The compounds can be tested for effectiveness as agonists and antagonists in the following test programme.

### Phase 1 - 3T3 Peptide Stimulated [³H] Thymidine Uptake Assay

Cell Culture. Stock cultures of Swiss 3T3 cells are grown in Dulbecco's Modified Eagles Medium (DMEM) supplemented with 10% fetal calf serum in humidified atmosphere of 10% CO₂/90% air at 37°C. For experimental use, the cells are seeded into 24-well cluster trays and used four days after the last change of medium. The cells are arrested in the G1/G0 phase of the cell cycle by changing to serum-free DMEM 24 hours prior to the thymidine uptake assay.

Assay of DNA Synthesis. The cells are washed twice with lml aliquots of DMEM (-serum) then incubated with DMEM (-serum), 0.5µM [methyl-³H] thymidine (20Ci/mmole, New England Nuclear); bombesin (3nM), and initially four concentrations of the test compounds (1, 10, 100, 1000nM) in a final volume of 1.0 ml. After 28, hours at 37°C. [methyl-³H] thymidine incorporation into acid-insoluble pools is assayed as follows. The cells are washed twice with ice-cold 0.9% NaCl (1ml aliquots), and acid soluble radioactivity is removed by a 30 min. (4°C) incubation with 5% trichloroacetic acid (TCA). The cultures are then washed once (1ml) with 95% ethanol and solubilized by a 30 min. incubation (1ml) with 0.1N NaOH. The solubilized material is transferred to vials containing 10ml ScintA (Packard), and the radioactivity is determined by liquid scintillation spectrometry.

### Phase 2 - Small Cell Carcinoma (SCLC) - Bombesin Stimulated [³H] Thymidine Uptake Assay

Cell Culture. Cultures of the human cell carcinoma cell line (NCI-H69) (obtained from the American Type Culture Association) are maintained in RPMI 1640 medium supplemented with 10% fetal calf serum in 10% CO₂/90% air at 37°C. Twenty-four hours prior to assay, the cells are washed with serum-free medium and seeded in 24-well cluster trays.

Assay of DNA Synthesis. Bombesin (1nM), 0.5µM [methyl-³H] thymidine (20 Ci/mmole, New England Nuclear), and four concentrations of the test compounds (1, 10, 100, 1000nM) are added to the cultures to achieve a final volume of 0.5 ml. After a 28 hr incubation at 37°C, the cells are collected onto GF/B glass fiber filters, and the DNA is precipitated with ice-cold TCA. [³H] thymidine incorporation into acid-insoluble fractions of DNA is determined by liquid scintillation spectrometry.

### Phase 3 - Peptide-Induced Pancreatitis

Male, Sprague-Dawley rats (250g) are used for these experiments. The test compound, or 0.9% NaCl is administered s.c. 15 min. prior to the bombesin injection. Bombesin injections are given s.c. at a dose of 10 µg/kg, and blood samples are obtained at 1 hr.30 min., 3hr. and 6hr. Plasma amylase concentration are determined by the Pantrak Amylase test.

### Phase 4- In Vitro Inhibition of [¹²⁵I] Gastrin Releasing Peptide (GRP) Binding to Bombesin Receptors

Membranes from various tissues (rat brain, rat pancreas, rat anterior pituitary, SCLC, 3T3 cells) are prepared by homogenization in 50mM TrisHCl containing 0.1% bovine serum albumin and 0.1mg/ml bacitracin followed by two centrifugations (39,000xgx15 min., 4°C) with an intermediate resuspension in fresh buffer. For assay, aliquots (0.8ml) are incubated with 0.5nM [¹²⁵I]GRP ('2000 Ci/mmol, Amersham Corp.) and various concentrations of the test compounds in a final volume of 0.5ml. After a 30 minute incubation at 4°C, the binding reaction is terminated by rapid filtration through Whatman GF/C filters that have been pre-soaked in 0.3% aqueous polethyleneimine to reduce the level of nonspecific binding. The filters and tubes are washed three times with 4ml aliquots of ice-cold buffer, and the radioactivity trapped on the filters is counted by gamma-spectrometry. Specific binding is defined as the total [¹²⁵I]GRP bound minus that bound in the presence of 1000nM bombesin.

### Phase 5- Inhibition of Gastrin Release

The stomachs of anesthetized rats are perfused with saline collected over 15 minute periods via pyloric cannulation while the test peptide is infused through the femoral vein for periods between 0 and 150 minutes.

### Phase 6- In Vivo Antitumor Activity

NCI-H69 small cell lung carcinoma cells were transplanted from in vitro culture by implanting each animal with the equivalent of 5 confluent 75 cm² tissue culture flasks in the right flank. In vitro NCI-H69 cells grow as a suspension of cellular aggregates. Therefore, no attempt was made to disaggregate the cell agglomerates by physical or chemical means. Tumor size was calculated as the average of two diameters, i.e., (length and width/2) mm.

### Results of Assays of Test Peptides

A number of analogs of bombesin or GRP, each containing a non-peptide bond can be synthesized and tested in one or more of the above-described Phase 1 - 6 assays; the results of Phase 1 and 2 tests are given in Tabie 1 attached hereto. Table I shows formulas for the non-peptide analogues and results of in vitro inhibition of [¹²⁵I]GRP binding to 3T3 fibroblast bombesin receptors, and bombesin-stimulated [³H]Thymidine uptake by cultured 3T3 cells. (3T3 GRP receptor and thymidine uptake data are expressed in IC50 (nM).) Table 1 also gives results for a non-peptide bond-containing analogue of one other naturally-occurring peptide, Neuromedin C, whose C-terminal seven amino acids are similar to those of bombesin and GRP. (In Table I, "Litorin" indicates a 9 residue peptide analog or its derivative, whereas "Neuromedin C" indicates a 10 residue analog or its derivative.)

In Table I, the position of the non-peptide bond is indicated by the position of the symbol ψ[CH₂NH]; i.e., ψ[CH₂NH] is always shown preceding the amino acid which, in that peptide, is bonded to the amino acid N-terminal to it via the non-peptide bond.

In Table I, it can be seen that BIM-26113 (D-Phe¹, Leu⁸ψ[CH₂NH]Leu⁹) and BIM-26114 (D-Nal¹, Leu⁸ψ[CH₂NH]Leu⁹) are active in the 3T3 GRP receptor binding and thymidine uptake assays. Most notably, BIM-26136 (D-Nal¹, Leu⁸ψ[CH₂NH]Phe⁹), which contains amino and carboxy terminal aromatic residues that are capable of forming a hydrophobic interaction, is the most potent analog.

Table I also shows that Neuromedin C analogue BIM-26107 is an antagonist when tested in the 3T3 GRP receptor and thymidine uptake assays.

Bombesin and Bombesin analogs have been shown to inhibit the effect of interleukin-2 (IL-2) (Fink et al., 1988, Klin. Wochenschr. 66, Suppl. 13, 273). Since IL-2 causes T lymphocytes to proliferate, it is possible that litorin antagonists may prevent the inhibitory effect of Bombesin or its analogs on IL-2. IL-2 stimulated lymphocytes are capable of effectively lysing small cell lung carcinoma cells in vitro. Although Bombesin antagonists have a direct antiproliferative effect on neoplastic tissues, they may also favor proliferation of lymphocytes having lytic activity for small cell lung carcinoma.

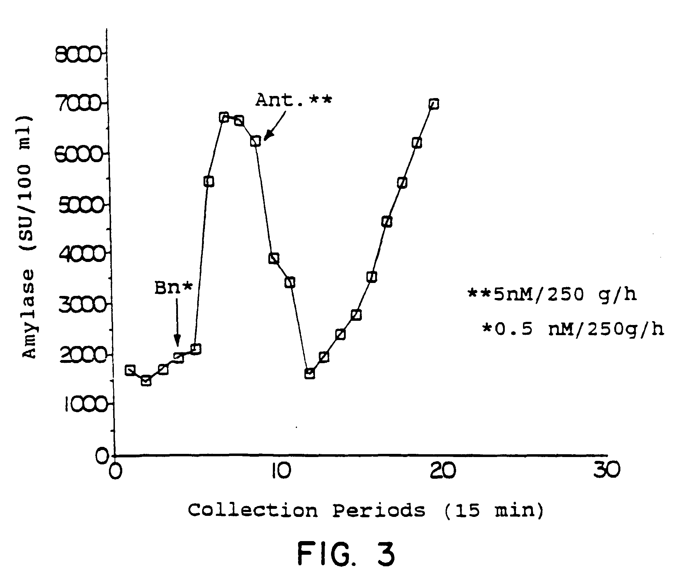

Fig. 3 shows the effect of the bombesin antagonist D-p-Cl-Phe-Gln-Trp-Ala-Val-Gly-His-Leuψ[CH2NH]Phe-NH2 on bombesin-stimulated amylase secretion in the rat. The results show that this analog is a potent antagonist; 5 nM of the analog can inhibit the secretion of anylase stimulated by 0.5 nM of bombesin for 150 minutes after bolus injection.

Our peptides may be administered to a mammal, particularly a human, in one of the traditional modes (e.g., orally, parenterally, transdermally, or transmucosally), in a sustained release formulation using a biodegradable biocompatible polymer, or by on-site delivery (e.g., in the case of anti-cancer bombesin to the lungs) using micelles, gels and liposomes.

Our bombesin antagonists are suitable for the treatment of all forms of cancer where bombesin-related substances act as autocrine or paracrine mitotic agents, particularly small-cell lung carcinoma. The peptides can also be used for the inhibition of gastric acid secretion and motility disorders of the GI tract, the symptomatic relief and/or treatment of exocrine pancreatic adenocarcinoma, and the restoration of appetite to cachexic patients. The peptides can be administered to a human patient in a dosage of 0.5 µg/kg/day to 5 mg/kg/day. For some forms of cancer, e.g., small cell lung carcinoma, the preferred dosage for curative treatment is 250mg/patient/day.

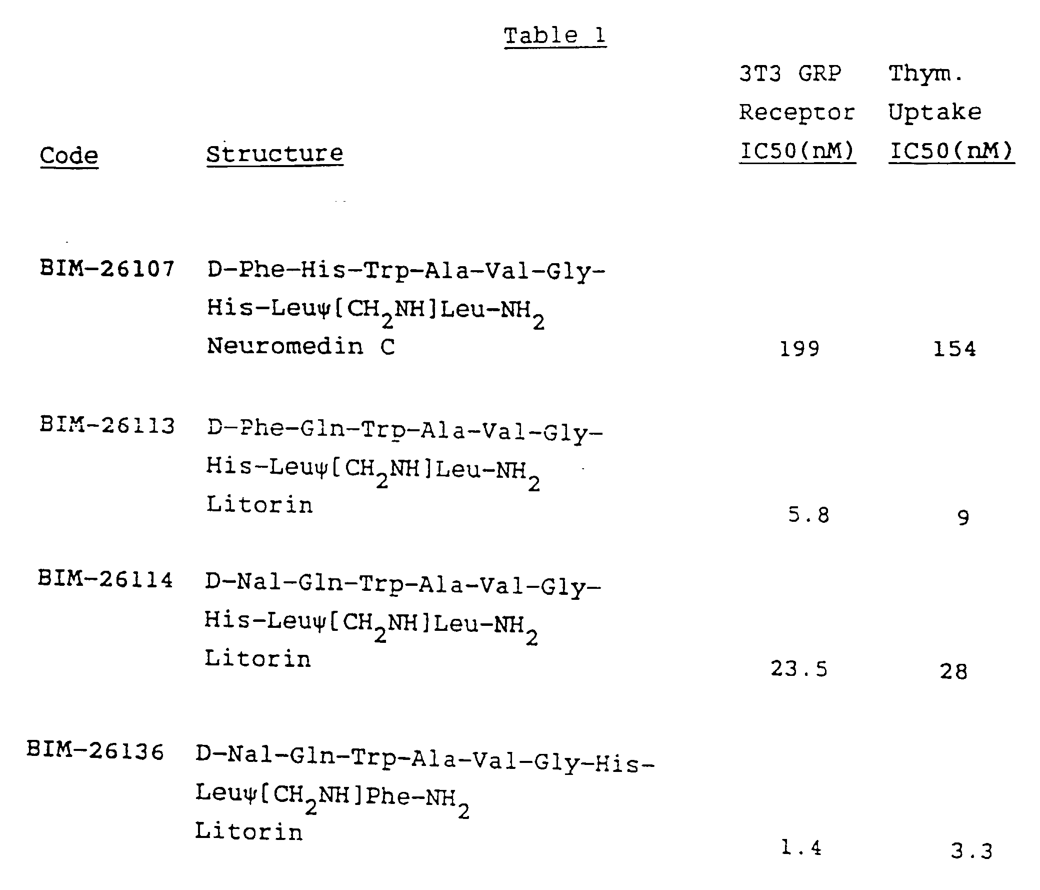

## Claims

1. A therapeutic peptide, said peptide comprising an analog of one of the following naturally occurring peptides terminating at the carboxy-terminus with a Met residue: (a) litorin, (b) neuromedin, (c) the ten amino acid carboxy-terminal region of mammalian gastrin releasing peptide, and (d) the ten amino acid carboxy-terminal region of amphibian bombesin; said peptide being of the formula: 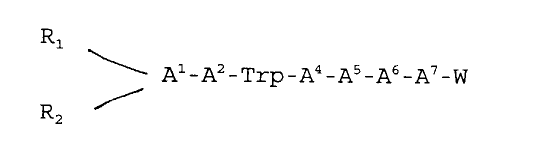 wherein
A¹ = the D-isomer of any of p-X-Phe (where X = F, Cl, Br, NO₂, OH, H or CH₃), Trp or β-Nal,
A² = Gln, His, 1-methyl-His, or 3-methyl-His,
A⁴ = Ala, Val, Gly, Leu, Ile, Nle or α-aminobutyric acid.
A⁵ = Gly, Ala, Leu, Ile, Nle, α-aminobutyric acid or Val,
A⁶ = Sar, Gly, or the D-isomer of any of Ala, Val, Leu or Ile,
A⁷ = 1-methyl-His, 3-methyl-His or His; and W is: 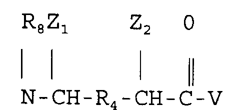 wherein R₄ is CH₂-NH, and each Z₁ and Z₂, independently, is the identifying group of any one of the amino acids Gly, Ala, Val, Leu, Ile, Ser, p-X-Phe (where X = H, F, C1, Br, NO₂, OH or CH₃), Trp, Met, Pro, HyPro, cyclohexyl-Ala, or cyclohexylmethyl and V is either OR₅ or 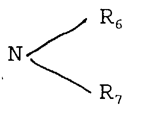 where each R₈, R₅, R₆, and R₇, independently, is H, lower alkyl, lower phenylalkyl, or lower naphthylalkyl; and wherein any asymmetric carbon atom of the group W can be R, S or a racemic mixture; and wherein each R₁ and R₂, independently, is H, C₁₋₁₂ alkyl, C₇₋₁₀ phenylalkyl, COE₁ (where E₁ is C₁₋₂₀ alkyl, C₃₋₂₀ alkenyl, C₃₋₂₀ alkynyl, phenyl, naphthyl, or C₇₋₁₀ phenylalkyl), and R₁ and R₂ are bonded to the N-terminal amino acid of said peptide; provided that when one of R₁ or R₂ is COE₁, the other must be H;
or a pharmaceutically acceptable salt thereof.

2. A therapeutic peptide of Claim 1 wherein,
A¹ = D-Phe, D-β-Nal, D-Cpa,
A² = Gln, His, 1-methyl-His, or 3-methyl-His;
A⁴ = Ala;
A⁵ = Val;
A⁶ = Sar, Gly or D-Ala;
A⁷ = His;
where Z₁ is cyclohexylmethyl or is the identifying group of Leu or Phe; and Z₂ is the identifying group of Met, Leu or Phe.

3. A therapeutic peptide according to Claim 1, wherein A¹ is D-β-Nal, each of Z₁ and Z₂, independently, is Leu or Phe.

4. A therapeutic peptide according to Claim 3 of the formula:
D-β-Nal-Gln-Trp-Ala-Val-Gly-His-Leuψ[CH₂NH]Leu-NH₂.

5. A therapeutic peptide according to Claim 3 of the formula:
D-β-Nal-Gln-Trp-Ala-Val-Gly-His-Leuψ[CH₂NH]Phe-NH₂.

6. A therapeutic peptide according to Claim 1, wherein R₄ is CH₂-NH, and said carbon atom bonded to Z₂ is of said R configuration.

7. A therapeutic peptide according to Claim 6 of the formula:
D-Phe-Gln-Trp-Ala-Val-Gly-His-Leuψ[CH₂NH]-D-Phe-NH₂.

8. A therapeutic peptide according to Claim 1, where A¹ is a D 8 A therapeutic peptide according to Claim 1, where A¹ is a D amino acid and V is OR₄.

9. A therapeutic peptide according to Claim 1 of the formula:
D-p-C1-Phe-Gln-Trp-Ala-Val-Gly-His-Leuψ[CH₂NH]Phe-NH₂.

10. A therapeutic peptide according to Claim 2 of the formula:
D-Phe-His-Trp-Ala-Val-Gly-His-Leuψ[CH₂NH]Leu-NH₂

11. A therapeutic peptide according to Claim 2 of the formula:
D-Phe-Gln-Trp-Ala-Val-Gly-His-Leuψ[CH₂NH]Leu-NH₂.

## Patentansprüche

1. Therapeutisch wirksames Peptid, das ein Analogon eines der folgenden natürlich vorkommenden am Carboxylterminal mit einem Met-Rest endenden Peptide (a) Litorin, (b) Neuromedin, (c) 10-Aminosäurecarboxylterminalbereich von Säugetiergastrin-Releasing-Peptid und (d) 10-Aminosäurecarboxylterminalbereich von Amphibienbombesin umfaßt und die folgende Formel aufweist: 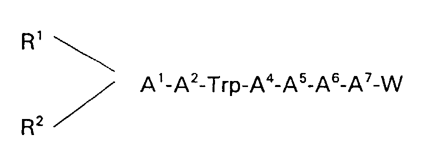 wobei:
A¹ = das D-lsomer von p-X-Phe (wobei X = F, Cl, Br, NO₂, OH, H oder CH₃), Trp oder β-Nal,
A² = Gln, His, 1-Methyl-His oder 3-Methyl-His,
A⁴ = Ala, Val, Gly, Leu, Ile, NIe oder α-Aminobuttersäure,
A⁵ = Gly, Ala, Leu, Ile, NIe, α-Aminobuttersäure oder Val,
A⁶ = Sar, Gly oder das D-lsomer von Ala, Val, Leu oder Ile,
A⁷ = 1-Methyl-His, 3-Methyl-His oder His ist und W 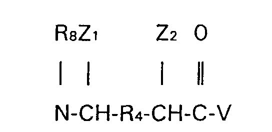 ist,
wobei R₄ CH₂-NH ist und Z₁ und Z₂ jeweils unabhängig voneinander die charakteristische Gruppe einer der Aminosäuren Gly, Ala, Val, Leu, Ile, Ser, p-X-Phe (wobei X = H, F, Cl, Br, NO₂, OH oder CH₃), Trp, Met, Pro, HyPro, Cyclohexyl-Ala oder Cyclohexylmethyl sind und V entweder OR₅ oder 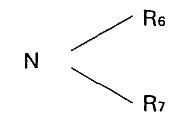 ist, wobei R₈, R₅, R₆ und R₇ jeweils unabhängig voneinander H, niederes Alkyl, niederes Phenylalkyl oder niederes Naphthylalkyl sind und wobei jedes asymmetrische Kohlenstoffatom der Gruppe W R, S oder ein razemisches Gemisch sein kann und wobei R₁ und R₂ jeweils unabhängig voneinander H, C₁₋₁₂Alkyl, C₇₋₁₀Phenylalkyl, COE₁ (wobei E₁ C₁₋₂₀Alkyl, C₃₋₂₀Alkenyl, C₃₋₂₀Alkylnyl, Phenyl, Naphthyl oder C₇₋₁₀Phenylalkyl ist) sind und R₁ und R₂ an die N-terminale Aminosäure des Peptids gebunden sind, vorausgesetzt, daß, wenn R₁ oder R₂ COE₁ ist, das andere von beiden H sein muß, oder ein pharmazeutisch nutzbares Salz davon.

2. Therapeutisch nutzbares Peptid nach Anspruch 1, bei dem
A¹ = D-Phe, D-β-Nal, D-Cpa,
A² = Gln, His, 1-Methyl-His oder 3-Methyl-His,
A⁴ = Ala,
A⁵ = Val,
A⁶ = Sar, Gly oder D-Ala,
A⁷ = His
ist,
wobei Z₁ Cyclohexylmethyl oder die charakteristische Gruppe von Leu oder Phe ist und Z₂ die charakteristische Gruppe von Met, Leu oder Phe ist.

3. Therapeutisch nutzbares Peptid nach Anspruch 1, wobei A¹ D-β-Nal ist und Z₁ und Z₂ jeweils unabhängig voneinander Leu oder Phe sind.

4. Therapeutisch nutzbares Peptid nach Anspruch 3 mit der Formel:
D-β-NaI-Gln-Trp-Ala-Val-Gly-His-Leuψ[CH₂NH]Leu-NH₂.

5. Therapeutisch nutzbares Peptid nach Anspruch 3 mit der Formel:
D-β-Nal-Gln-Trp-Ala-Val-Gly-His-Leuψ[CH₂NH]Phe-NH₂.

6. Therapeutisch nutzbares Peptid nach Anspruch 1, wobei R₄ CH₂-NH ist und das an Z₂ gebundene Kohlenstoffatom die R-Konfiguration aufweist.

7. Therapeutisch nutzbares Peptid nach Anspruch 6 mit der Formel:
D-Phe-Gln-Trp-Ala-Val-Gly-His-Leuψ[CH₂NH]-D-Phe-NH₂.

8. Therapeutisch nutzbares Peptid nach Anspruch 1, wobei A¹ eine D-Aminosäure und V OR₄ ist.

9. Therapeutisch nutzbares Peptid nach Anspruch 1 mit der Formel:
D-p-Cl-Phe-Gln-Trp-Ala-Val-Gly-His-Leuψ[CH₂NH]Phe-NH₂.

10. Therapeutisch nutzbares Peptid nach Anspruch 2 mit der Formel:
D-Phe-His-Trp-Ala-Val-Gly-His-Leuψ[CH₂NH]Leu-NH₂.

11. Therapeutisch nutzbares Peptid nach Anspruch 2 mit der Formel:
D-Phe-Gln-Trp-Ala-Val-Gly-His-Leuψ[CH₂NH]-Leu-NH₂.

## Revendications

1. Peptide thérapeutique, ledit peptide comprenant un analogue d'un des peptides naturels suivants se terminant à l'extrémité carboxyle terminale par un résidu Met : (a) litorine, (b) neuromédine, (c) région carboxyle terminale de 10 acides aminés du peptide libérant la gastrine mammalienne et (d) région carboxyle terminale de 10 acides aminés de la bombésine de batracien ; ledit peptide ayant la formule : 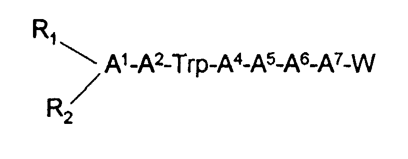 où
A¹ = isomère D de p-X-Phe (où X = F, Cl, Br, NO₂, OH, H ou CH₃), Trp ou β-Nal,
A² = Gin, His, 1-méthyl-His ou 3-méthyl-His,
A⁴ = Ala, Val, Gly, Leu, Ile, NIe ou acide α-aminobutyrique,
A⁵ = Gly, Ala, Leu, Ile, NIe, acide α-aminobutyrique ou Val,
A⁶ = Sar, Gly ou l'isomère D de Ala, Val, Leu ou Ile,
A⁷ = 1-méthyl-His, 3-méthyl-His ou His ; et W est : 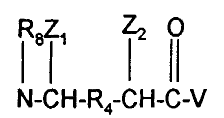 où R₄ est CH₂-NH et Z₁ et Z₂, indépendamment, sont chacun le groupe identificateur d'un quelconque des acides aminés Gly, Ala, Val, Leu, Ile, Ser, p-X-Phe (où X = H, F, Cl, Br, NO₂, OH ou CH₃), Trp, Met, Pro, HyPro, cyclohexyl-Ala ou cyclohexylméthyl et V est soit OR₅, soit 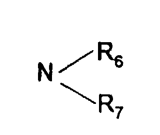 où R₈, R₅, R₆ et R₇ sont chacun, indépendamment, H, un alkyle inférieur, un phénylalkyle inférieur ou un naphtylalkyle inférieur ; et où tout atome de carbone asymétrique du groupe W peut être R, S ou un mélange racémique ; et où R₁ et R₂, indépendamment, sont chacun H, un alkyle en C₁₋₁₂, un phénylalkyle en C₇₋₁₀, COE₁ (où E₁ est un alkyle en C₁₋₂₀, un alcényle en C₃₋₂₀, un alcynyle en C₃₋₂₀, un phényle, un naphtyle ou un phénylalkyle en C₇₋₁₀) et R₁ et R₂ sont liés à l'acide aminé N-terminal dudit peptide ; pour autant que lorsque R₁ ou R₂ est COE₁, l'autre doit être H ;
ou un sel pharmaceutiquement acceptable de ce dernier.

2. Peptide thérapeutique selon la revendication 1, où
A¹ = D-Phe, D-β-Nal, D-Cpa,
A² = Gln, His, 1-méthyl-His ou 3-méthyl-His,
A⁴ = Ala ;
A⁵ = Val ;
A⁶ = Sar, Gly ou D-Ala ;
A⁷ = His ;
où Z₁ est un cyclohexylméthyle ou est le groupe identificateur de Leu ou Phe ; et Z₂ est le groupe identificateur de Met, Leu ou Phe.

3. Peptide thérapeutique selon la revendication 1, où A¹ est D-β-Nal, Z₁ et Z₂, indépendamment, sont chacun Leu ou Phe.

4. Peptide thérapeutique selon la revendication 3, de formule : D-β-Nal-Gln-Trp-Ala-Val-Gly-His-Leuψ[CH₂NH]Leu-NH₂.

5. Peptide thérapeutique selon la revendication 3, de formule : D-β-Nal-Gln-Trp-Ala-Val-Gly-His-Leuψ[CH₂NH]Phe-NH₂.

6. Peptide thérapeutique selon la revendication 1, où R₄ est CH₂-NH et ledit atome de carbone lié à Z₂ est de ladite configuration R.

7. Peptide thérapeutique selon la revendication 6, de formule : D-Phe-Gln-Trp-Ala-Val-Gly-His-Leuψ[CH₂NH]-D-Phe-NH₂.

8. Peptide thérapeutique selon la revendication 1, où A¹ est un acide aminé à la configuration D et V est OR₄.

9. Peptide thérapeutique selon la revendication 1, de formule : D-p-Cl-Phe-Gln-Trp-Ala-Val-Gly-His-Leuψ[CH₂NH]Phe-NH₂.

10. Peptide thérapeutique selon la revendication 2, de formule : D-Phe-His-Trp-Ala-Val-Gly-His-Leuψ[CH₂NH]Leu-NH₂.

11. Peptide thérapeutique selon la revendication 2, de formule : D-Phe-Gln-Trp-Ala-Val-Gly-His-Leuψ[CH₂NH]Leu-NH₂.
